# EUROPEAN PATENT APPLICATION

(11) **EP 0 857 696 A2**
(43) Date of publication of application: **12.08.1998**
(21) Application number: 97303634.6
(22) Date of filing: 29.05.1997
(51) Int. Cl.: C02F 3/02, C02F 11/02, C02F 1/52, C02F 3/34, G01N 33/18

(54) **A system for treating sewage-water and a method for treating sewage-water using the same**

(30) Priority: 05.02.1997 KR 9703588
(71) Applicant: Haekang Engineering & Construction Co., Ltd., Keumjung-ku, Pusan (KR)
(72) Inventor: Gi-Moon, Sung, Inchen, Kyunggi Province (KR); Sung-Ki, Baek, Songpa-ku, Seoul (KR); Choi Yong-Su, Nowon-Ku, Seoul (KR)
(74) Representative: Style, Kelda Camilla Karen

(57) **Abstract**

This invention relates to a system for treating sewage-water and a method for treating sewage-water using the same, being characterized in that for treating the sewage-water using the standard activated sludge method, Bacillus, one of facultative filamentous bacteria, is cultivated and predominantly occupied in high concentration from both aeration tank and aerobic sludge digestion tank and then, the flowing sewage-water may be treated by said Bacillus cultivated in a higher concentration. Further, a supervisory monitoring system is utilized for accurately observing the microorganisms in the tanks and offensive-odoring gases generated from the first sewage-water treatment process are disintegrated in the aeration tank for elimination thereof, thus making it available to eliminate nitrogen and phosphorus including organic substances contained in the sewage-water and to significantly reduce the volume of sewage-water generated.

## Description

The invention relates to a system for treating sewage-water and a method for treating sewage-water using the same.

The standard activated sludge processing method, which has been most widely used for treating the sewage-water, comprises the following five process steps of:
I) Pretreatment process for eliminating sand or adulteration contained in the sewage-water;
ii) First treatment process for precipitating and eliminating suspended solid contained in the sewage-water using a plain sedimentation;
iii) Second treatment process for introducing the supernatant formed during the first treatment process in an aeration tank so as to treat the supernatant;
iv) Exhaust water disinfecting process for disinfecting the water, which is treated by the second treatment process, prior to exhausting the water; and
v) Sludge treatment process for dehydrating sludge formed by the first and second treatment processes and discharging the dehydrated sludge.

Of the aforementioned five basic process steps, three treatment process steps (e.g., pretreatment, first treatment process and disinfecting process) are similarly used as in the majority of sewage disposal plants, while the method of treating sewage-water is classified the selection on the second and sludge treatment process steps. Namely, based on the individual characteristic of the second and sludge treatment process steps or proper combination of them, the characteristics of the sewage-water treating method is actually determined.

In general, the standard activated sludge method and its modified method are mainly used as the second treatment process. In case of the sewage-water treatment facilities having relatively smaller capacity, some modified method of activated sludge are being adopted. However, if the sewage-water treatment facilities have a larger capacity, the standard activated sludge method is being used. Currently, about 80% of sewage disposal plants have predominantly adopted the standard activated sludge method around the world.

The standard activated sludge method has been probably recognized as the most recommended process among the conventional applicable processes but such process has also the following shortcomings:
I) With a considerable amount of sludge generated thus, poor dewaterbility in sludge leads to extremely poor dehydration efficiency in the sludge treatment process. Also, even though the elimination efficiency of organic substances proves to be remarkable, the standard activated sludge method cannot eliminate nitrogen and phosphorus.
ii) In the event that any operational malfunctions such as bulking during the sewage-water treatment occur, it takes more than one month until the normal operation is resumed. Thus the recovery period of time is lengthened.
iii) To comply with any offensive-odoring gas generated from the sewage-water treatment process or bad odors in sludges generated from the first and 2nd treatment process steps, the following problems have been raised: - In case of bad odors due to high concentration, some substances generating bad odors should be incinerated or after washing them with chemicals, activated carbon should be additionally used for adsorption and deodorization; - In case of bad odors generated by medium- or low-concentration, some substances generating bad odor should be washed with some chemicals or water and then activated carbon should be also used for adsorption, or their deodorization should be made via soil deodorization method, biological filtration method, etc. Under such circumstances, separate deodorization facilities such as an incinerator for elimination of bad-odor substances including scrubbing tower or adsorption tower are inevitably required and if this being the case, their heavy operation and maintenance costs should be incurred.
iv) The system for treating sewage-water is influenced by various factors but since these factors are computed to meet the physicochemical conditions for proper operation of a system for treating sewage-water, sophisticated level of technology should be required for the efficient operation of such system via suitable controlling method but this is considered to be a tough assignment to experts.
v) To eliminate the contaminants in the most effective manner, therefore, an optimum phase of microorganisms and through continuous observation, the microorganisms should be kept in an optimum level. Nevertheless, the conventional systems for treating the sewage-water have recognized some disadvantages in that a laboratory is equipped with a microscope to observe protozoas simply for ascertaining their presence irregularly, thus making it unavailable to operate the system based on the accurate assessment of bacteria, the most important factor among other things.

To be free from the above mentioned shortcomings, an object of certain embodiments of this invention is to provide a novel system for treating sewage-water.

Under the system for treating sewage-water using the standard activated sludge method, Bacillus is selectively cultivated and occupied predominantly in sewage-water, and aeration tank and aerobic sludge digestion tank where sludges generated from the sewage-water are treated; Continuous observation on microorganism such as protozoa, etc., including bacteria such as Bacillus in the aeration tank and aerobic sludge digestion tank using a supervisory monitoring system; offensive-odoring gases discharged to the aeration tank may be intaked or dissolved by Bacillus for deodorization thereof so that nitrogen and phosphorus contained in the sewage-water may be eliminated more effectively; The results observed by a supervisory monitoring system may be utilized for controlling various influential factors; some bad odors generated in the process for treating the sewage-water may be effectively eliminated without any separate facilities.

Further, another aim of embodiments of this invention is to provide a novel method for treating sewage-water. Under the method for treating sewage-water using the standard activated sludge method, sludges generated from the sewage-water are disintegrated and treated by Bacillus cultivated and predominantly occupied from aeration tank and aerobic sludge digestion tank so as to a) effectively eliminate nitrogen and phosphorus as well as organic substances, b) eliminate some bad odors generated from the aeration tank, and c) reduce the generation of sludge.

For a better understanding of the present invention and as to how the same may be carried into effect, reference will now be made to the accompanying drawings in which:

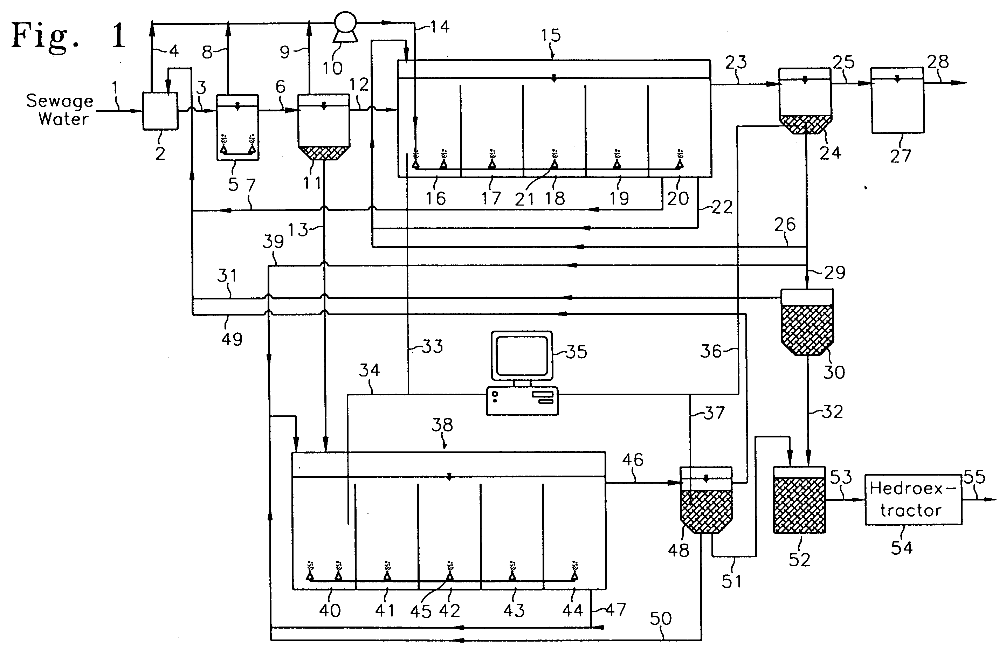

Fig. 1 is a flow chart showing a system for treating sewage-water and a method for treating sewage-water using the same embodying this invention.

An embodiment of this invention is described in more detail by referring to the attached drawings as set forth hereunder.

Fig. 1 is a flowchart of a system for treating sewage-water and a method for treating sewage-water embodying this invention. The system for treating sewage-water embodying this invention comprises thirteen main sections, i.e. pretreatment facilities 2, a flow control tank 5, a primary sedimentation tank 11, an aeration tank 15, a final sedimentation tank 24, an aerobic sludge digestion tank 38, a sedimentation tank 48 for the aerobic sludge digestion tank 38, chemical flocculent settling facilities 27, a sludge thickener 30, a sludge storage tank 52, a supervisory monitoring system 35 and a sludge hydroextractor 54, as shown in Fig. 1.

The pretreatment facilities 2 is provided to eliminate adulteration, microsome, or uncorrosive inorganic substances contained in sewage-water supplied from the conduit. The flow control tank 5 is provided to control the supply of water 3 in predetermined amount, so produced from the pretreatment facilities 2. In the primary sedimentation tank 11, water 6, so supplied from the flow control tank 5, is settled for a certain period of time and suspended solids of more than 5µ are precipitated.

Also, in the aeration tank 15, water 12, so supplied from the primary sedimentation tank 11, is aerially treated and at the same time, bacillus is selectively cultivated and occupied predominantly. In the final sedimentation tank 24, water 23, so supplied from the aeration tank 15, is settled for a certain period of time and its solid/liquid separation occurs by gravity. In the aerobic sludge digestion tank 38, raw-sludge 13, so precipitated from the primary sedimentation tank 11, including sludge returned after being precipitated in the final sedimentation tank 24, are supplied for aerial treatment and at the same time, bacillus is selectively cultivated and occupied predominantly. The sedimentation tank 48 used for the aerobic sludge digestion tank 38, water 46, so discharged from the aerobic sludge digestion tank 38, is supplied and settled for a certain period of time and the precipitation of sludge contained in water 46 occurs. In the chemical flocculent settling facilities 27, water 25, so supplied from the sedimentation tank 24, is adequately treated to meet the discharge standards. The sludge thickener 30 is provided where remaining excess sludge 29 unreturned after precipitation in the final sedimentation tank 24 is concentrated. The sludge storage tank 52 is provided where remaining excess sludge 29 unreturned after precipitation in the sedimentation tank 48 for aerobic sludge digestion tank as well as sludge 32 concentrated from sludge thickener 30 are reserved. The supervisory monitoring system 35 is provided where some microbes such as bacteria, protozoa, etc. present in the aeration tank 15, final sedimentation tank 24, aerobic sludge digestion tank 38 and sedimentation tank 48 for the aerobic sludge digestion tank are under closer observation in order for its results to be utilized for controlling various influencing factors. The sludge hydroextractor 54 is provided where sludge 53, so discharged from the sludge storage tank 52, is dehydrated.

In the above described construction, the aeration tank 15 is separated and partitioned into four chambers, i.e., first chamber 16, second chamber 17, third chamber 18 and fourth chamber 19, each of which has an aerator 21. The aeration tank 15 at the discharging part is provided with an inner circulation chamber 20 which is designed to internally circulate the sludge into the first chamber 16, while playing a role as simple sedimentation chamber.

Meantime, in the system for treating sewage-water of this invention, the aerobic sludge digestion tank 38 is, as shown in the aeration tank 15, separated and partitioned into four chambers where each aerator 45 is provided, i.e., first chamber 40, second chamber 41, third chamber 42 and fourth chamber 43. The aerobic sludge digestion tank 38 is provided with an inner circulation chamber 44 which is designed to internally circulate the sludge into the first chamber 40.

The inner circulation chamber 20 of the aeration tank 15 is constructed in order that to treated amount of flowing water, some mixing solution in certain ratio may be circulated into the pretreatment facilities 2 and sludge-separated liquid 31, so generated from the sludge thickener 30 and water 49, so produced from the sedimentation tank 48 for aerobic sludge digestion tank is resupplied into the pretreatment facilities 2.

Further, the supervisory monitoring system 35 comprises five main installations, i.e., a phase contrast microscope, a camera, a video set, a monitor and a printer. The Phase contrast microscope is designed to observe microorganisms and a camera to photograph some microorganisms observed under the phase contrast microscope. The video set is designed to investigate the microorganisms photographed by the camera and the monitor to display microorganisms related information reproduced by the video set. The printer is provided to print microorganisms related information displayed on the monitor.

Meantime, offensive-odoring gases 4, 8 and 9, so generated from the pretreatment facilities 2, flow control tank 5 and primary sedimentation tank 11, are inhaled and piled into a single exhaust line independently provided and supplied them into the first chamber 16 in the aeration tank 15 by ventilation of air-blower 10 provided on the exhaust line.

The method for treating sewage-water using the above mentioned system for treating sewage-water of this invention will be described hereinafter.

The method for treating sewage-water, as illustrated in Fig. 1, is applied in using pretreatment facilities 2, flow control tank 5, primary sedimentation tank 11, aeration tank 15, final sedimentation tank 24, aerobic sludge digestion tank 38, sedimentation tank for aerobic sludge digestion tank 48, sludge thickener 30, sludge storage tank 52 and sludge hydroextractor 54. The amounts of total air, being charged to the aeration tank 15 and aerobic sludge digestion tank 38 are aerated by gradation to the first chambers 16 and 40 and remaining second chambers 17 and 41, third chambers 18 and 42 and fourth chambers 19 and 43 in a certain ratio, respectively. At the same time, essential nutrients for cultivation of Bacillus are given into the aeration tank 15 and aerobic sludge digestion tank 38, and the internal circulation of sludge is made available between each inner circulation chamber 20 and 44 and first chamber 16 and 40, so formed in both the aeration tank 15 and aerobic sludge digestion tank 38. Also, some sludge 26 deposited from final sedimentation tank 24 is returned and supplied to the first chamber 16 of the aeration tank 15, while some sludges 50 and 39 deposited from sedimentation tank for aerobic sludge digestion tank 48 and final sedimentation tank 24, respectively, is returned and supplied to the first chamber 40 of the aerobic sludge digestion tank 38. In this manner, cultivated bacillus may be predominantly occupied through the following growth steps of Bacillus, i.e., "filamentous fungus-spores-germination", while a supervisory monitoring system 35 is used to continuously observe the growth steps of Bacillus in aeration tank 15, final sedimentation tank 24, aerobic sludge digestion tank 38 and sedimentation tank for aerobic sludge digestion tank 48.

According to the method for treating sewage-water embodying this invention, important nutrients for cultivation of Bacillus include silicide and magnesium compound in an effort to facilitate the proliferation of fungus and induce its spore process; the dose of the silicide is 0.01∼0.1kg/kg·BOD, while that of the magnesium compound is 0.005∼0.05kg/kg·BOD, being a half dose of silicide.

According to the method for treating sewage-water of this invention, 60∼80% (preferably 70%) of the total air amount supplied to each aeration tank 15 and aerobic sludge digestion tank 38 should be intensively given to each of the first chamber 16 40, so formed in the aeration tank 15 and aerobic sludge digestion tank 38, while 20∼40% of the total air amount should be aerated in small amount to the remaining second chambers 17, 41, third chambers 18, 42 and fourth chambers 19 and 43 based on tapered allocation.

As such, in case where 60∼80% of the total air amount should be intensively given to each of the first chambers 16 and 40, so formed in the aeration tank 15 and aerobic sludge digestion tank 38, while 20∼40% of the total air amount should be aerated in small amount to the remaining second chambers 17 and 41, third chamber 18, 42 and fourth chambers 19, 43 based on tapered allocation, the concentration of dissolved oxygen, when the air is transferred from the first chambers 16, 40 to fourth chambers 19, 43, is tapered and thus the interior of both aeration tank 15 and aerobic sludge digestion tank 38 is maintained as facultative state. Under such conditions, the growth of aerobic germs, which have been mainly used in the convention system for treating sewage-water, is inhibited and some Bacillus with larger resistance may be selectively cutivated and predominantly occupied.

Bacillus, so cultivated and predominantly occupied by the above action, is rapidly proliferating with the supply of silicide and magnesium compound, essential nutrients for cultivation, and the concentration becomes high. Under a high concentration, the Bacillus contacts with organic substances, nitrogen, and phosphorus contained in the sewage-water and its generated sludges supplied into the aeration tank 15 and aerobic sludge digestion tank 38 and thus some substances such as nitrogen, phosphorus and organic substances are eliminated by its metabolic action. Under the method for treating sewage-water embodying this invention, the return rate of sludge in aeration tank internal circulation 22 is 100∼300% to treated amount of flowing water. Now that the rate of sludge returned from the outside to the aeration tank 15 is 50∼200% to treated amount of flowing water plus the returned rate from final sedimentation tank sludge 26 to treated amount of flowing water. Thus the total returned rate of sludge supplied to the first chamber 16 of aeration tank 15 is 150∼500% to treated amount of flowing water.

Meantime, the sludge return rate in aerobic sludge digestion tank internal circulation 47 is 200∼300% to treated amount of flowing water. In case of the sludgerate returned from the outside to aerobic sludge digestion tank 38, the sludge return rate from precipitation tank for aerobic sludge digestion tank is 100% to treated amount of flowing water and the rate supplied from final sedimentation tank 39 is 200∼300%. Thus the total sludge return rate supplied to the first chamber 40 of aerobic sludge digestion tank 38 is 500∼700% to treated amount of flowing water.

As described above, the sludge return rate supplied to first chamber 16 of aeration tank 15 and first chamber 40 of aerobic sludge digestion tank 38 is properly controlled by the following factors, i.e., phase of microorganisms within aeration tank 15 and aerobic sludge digestion tank 38, temperature, pH, amount of dissolved oxygen, etc.

Under such sludge return process, remaining excess sludge 29 unreturned to aeration tank 15 and aerobic sludge digestion tank 38 from the final sedimentation tank 24 is transferred to sludge thickener 30 for concentration thereof and then supplied to sludge storage tank 52. During this process, sludge-separated liquid 31 separated from the sludge thickener 30 is supplied to pretreatment facilities 2 for retreatment, and remaining excess sludge 51 of the sedimentation tank 48 for aerobic sludge digestion tank unreturned to aerobic sludge digestion tank 38 is transferred to sludge storage tank 52 for controlling. Then, a certain amount of the sludge is dehydrated from sludge hydroextractor 54 and discharged in the form of sludge cake 55 for final use as compost, etc.

Under the method for treating sewage-water embodying this invention, in order for Bacillus to be grown from pretreatment facilities 2 and to eliminate offensive-odoring substances before the sewage-water is supplied to aeration tank 15, the sludge of aeration tank 7 having the returned rate 1∼3% to treated amount of flowing water is returned from inner circulation chamber 20 of the aeration tank circulation water 15 to pretreatment facilities 2.

Further, offensive-odoring gases 4, 8 and 9 generated from the pretreatment facilities 2, flow control tank 5 and primary sedimentation tank 11 are inhaled and collected into a single exhaust line. With ventilation of air-blower 10, these gases are supplied into first chamber 16 of aeration tank 15 where Bacillus is cultivated and predominantly occupied in a higher concentration. Within such chamber, these gases are disintegrated and eliminated by the Bacillus. In the process of eliminating bad odors, nitrogen substances in the sewage-water generating peculiar bad odors is dissolved into treatment solution of aeration tank 15 and at the same time, these bad odors are disintegrated and eliminated by the metabolic action of Bacillus being cultivated and predominantly occupied in aeration tank 15. Also, hydrogen sulfide generating peculiar bad odors is changed into sulfur oxide without any odor, since the interior of aeration tank 15 maintains aerobic state in part.

In this manner, related installations (e.g., aeration tank 15), aerobic sludge digestion tank 38, sludge thickener 30, sludge storage tank 52, sludge hydroextractor 54 or others do not have little bad odors.

Under the method for treating sewage-water of this invention, raw-sludge 13 precipitated from the primary sedimentation tank 11 is immediately transferred to aerobic sludge digestion tank 38 and digested aerobically by Bacillus.

In embodiments of this invention, the flow control tank 5, Chemical flocculent settling facilities 27, sludge thickener 30 and aerobic sludge digestion tank 38 may be added or exempted from the process based on the scale of sewage-water treatment.

Meantime, a non-described code 14 indicated in the Figure is a offensive odor in high concentration supplied by air-blower 10 from first chamber 16 in aeration tank 15 ; a non-described code 28 is a discharged water; other non-described codes 33, 34, 36 and 37 are indicative lines showing the regular collection of treatment solutions within aeration tank 15, aerobic sludge digestion tank 38, final sedimentation tank 24 and sedimentation tank 37 for aerobic sludge digestion tank, respectively. The state of microbes such as protozoas and bacteria including Bacillus in the treatment solutions regularly collected is observed by a monitor formulating supervisory monitoring system 35 and its results is utilized for controlling various influential factors.

The process steps for treating sewage-water based on the method for treating sewage-water will be described hereinafter.

As illustrated in Fig. 1, when sewage-water is supplied via conduit 1, some adulteration. microsome, or uncorrosive inorganic substances contained in sewage-water is eliminated from pretreatment facilities 2 provided with screen and detritus facilities and transferred to flow control tank 5. Via the flow control tank 5, a certain amount of sewage-water is supplied to primary sedimentation tank 11 where suspended solids of more than 5µ are precipitated for elimination thereof and then supplied to aeration tank 15 for aeration treatment. At this time, the sewage-water supplied to aeration tank 15 is contacted with Bacillus cultivated in high concentration in the first chamber 16 and then organic substances, nitrogen and phosphorus contained in the sewage-water are eliminated by the metabolism of Bacillus. Such actions are continuously effective from second chamber 17, third chamber 18 to fourth chamber 19.

In the aeration treatment process of sewage-water as aforementioned, the total returned rate for sludge including internal circulation 22 should be adjusted to 150∼500%. The retention time of microorganism is further extended with increase of the fungus amount. At that same time, 60-80% of the total air amount should be intensively given to each of the first chamber 16, so formed in the aeration tank 15, while 20∼40% of the total air amount should be aerated in small amount to the remaining chambers 17, 18 and 19 based on uniformity allocation, the air transfer from the first chamber 16 to fourth chamber 19 tapers the concentration of dissolved oxygen. Now that the interior of in aeration tank 15 is facultative, the growth of aerobic microbes becomes inhibitive, while Bacillus, Gram-positive facultative bacteria with large resistance, is cultivated and predominantly occupied in the aeration tank 15. The Bacillus is rapidly proliferated by a certain dose of silicide and magnesium compound supplied to the first chamber 16 of aeration tank 15.

In an effort to increase the number of Bacillus necessary for its predominantly occupied cultivation in the aeration tank 15, some sludge of final sedimentation tank 24 as well as aeration tank internal circulation 22 are returned into aeration tank 15 at the sludge return rate of 50∼200% to treated amount of flowing water. Further, in order for Bacillus to be grown from pretreatment facilities 2, 1∼3% aeration tank circulation water 7 to treated amount of flowing water is transferred from the inner circulation chamber 20 to pretreatment facilities 2.

The water 23, so aerially treated, is supplied to final sedimentation tank 24, settled, and separated in the form of solid/liquid by gravity. Then, supernatant 25 is discharged as it is or if there is a necessity that phosphorus, etc. contained in the supernatant should be further treated in a lower concentration, advanced sewage-water treatment on such supernatant is performed for discharge 28 thereof.

Meantime, raw-sludge 13 deposited from primary sedimentation tank 11 is supplied into aerobic sludge digestion tank 38 and treated aerially via first chamber 40, second chamber 41, third chamber 42, fourth chamber 43 and inner circulation chamber 44 in aerobic sludge digestion tank 38. During this process, organic substance, nitrogen and phosphorus contained in raw-sludge 13 may be eliminated by the metabolic action of Bacillus in predominantly occupied cultivation according to the same principle as the above aeration tank 15.

In an effort to increase the number of Bacillus necessary for its predominantly occupied cultivation in the aerobic sludge digestion tank 38, aerobic sludge digestion tank internal circulation 47 is performed at the sludge return rate of 200∼300% to treated amount of flowing water. At the same time, some sludge of sedimentation tank for aerobic sludge digestion tank 48 is returned to the first chamber 40 of aerobic sludge digestion tank 38 at the sludge return rate of 100% to treated amount of flowing water, while some sludge of the final sedimentation tank 24 is returned to the first chamber 40 of aerobic sludge digestion tank 38 at the returned rate of 200∼300% to treated amount of flowing water.

Under such aeration treatment process, the total return rate for sludge including internal circulation 47 should be adjusted to 500∼700%. The retention time of microorganism is further extended with increase of the fungus amount. At that same time, 60-80% (preferably 70%) of the total air amount should be intensively given to each of the first chamber 40, so formed in the aerobic sludge digestion tank 38, while the remaining air amount should be aerated in small amount to the remaining chambers 41 42 43 based on tapered allocation, the air transfer from the first chamber 40 to fourth chamber 43 tapers the concentration of dissolved oxygen. Now that the interior of in aeration tank 38 is facultative, the growth of aerobic microbes becomes inhibitive, while Bacillus, Gram-positive facultative bacteria with large resistance, is cultivated and predominantly occupied in the aerobic sludge digestion tank 38. The Bacillus is rapidly proliferated by a certain dose of silicide and magnesium compound supplied to the first chamber 40 of aerobic sludge digestion tank 38.

To ensure the survival environment of Bacillus, a target microorganism for the predominant occupation, it is necessary that a small amount of sludge from an initial process step should be returned from both aeration tank 15 and inner circulation chambers 20 44 of aerobic sludge digestion tank 38 to first chambers 16 40.

Meantime, remaining excess sludge 29 unreturned to aeration tank 15 and aerobic sludge digestion tank 38 from the final sedimentation tank 24 is transferred to sludge thickener 30 for concentration thereof and then supplied to sludge storage tank 52.

During this process, sludge-separated liquid 31 separated from the sludge thickener 30 is supplied to pretreatment facilities 2 for retreatment, and remaining excess sludge 51 of the sedimentation tank 48 for aerobic sludge digestion tank unreturned to aerobic sludge digestion tank 38 is transferred to sludge storage tank 52 for controlling. Then, a certain amount of the sludge is dehydrated from sludge hydroextractor 54 and discharged in the form of sludge cake 55.

Under such sludge treatment process, sludge-separated liquid 31 discharged from the sludge thickener 30 and water 49 discharged from the sedimentation tank for aerobic sludge digestion tank 48 are resupplied to pretreatment facilities 2 for retreatment thereof.

Meantime, offensive-odoring gases 4, 8 and 9 generated from the pretreatment facilities 2, flow control tank 5 and primary sedimentation tank 11 are inhaled and collected into a single exhaust line. With ventilation of air-blower 10, these gases are supplied into first chamber 16 of aeration tank 15. Nitrogen, a main substance generating the offensive-odoring gas is dissolved with treatment solution in first chamber 16 and at the same time, the gas is disintegrated by the metabolic action of Bacillus for deodorization. Further, now that the interior of aeration tank 15 maintains aerobic state in part, hydrogen sulfide is changed into sulfur oxide, thus contributing to deodorization EXAMPLE 1

As shown in the following table 1, appearance of flowing sewage and raw-sludge is illustrated as an example of treating the sewage at 6.000 m³ per day.

**[TABLE 1]**

| | Suspended Solid (SS) | Biochemical Oxygen Demand (BOD) | Chemical Oxygen Demand (COD) | Total Nitrogen (T-N) | Total Phosphorus (T-P) |
|---|---|---|---|---|---|
| Sewage-water | 180 | 200 | 160 | 40 | 8 |
| Raw-Sludge | 40,000 | 6,700 | 4,690 | 330 | 160 |

As revealed in the above table 1, the sewage supplied from flow control tank 5 was precipitated in primary sedimentation tank 11. Then, after treatment in the active sludge process, the sewage was further under chemical flocculent settling treatment process for discharge thereof, if necessary. Further, raw-sludge 13 precipitated from primary sedimentation tank 11 was treated in aerobic sludge digestion tank 38 and its separated liquid was retreated from pretreatment facilities 2. Then, excess sludge 51, so generated, was dehydrated in sludge hydroextractor 54 and finally disposed in the form of sludge cake 55. The operation conditions on aeration tank 15 and aerobic sludge digestion tank 38 is described in the following table 2.

**[TABLE 2]**

| | Amount of Flowing Water (m³/d) | Tank Capacity (m³) | Retention time (hr) | Space Loading of BOD (kg/m³· day) | MLSS loading of BOD (kg/kg· day) | Returned ratio of sludge (vs. treating amount %) | MLSS (mg/l) |
|---|---|---|---|---|---|---|---|
| Aeration tank | 6,000 | 2,000 | 8 | 0.6 | 0.2 | 150-500 | 3,000 |
| Digestion tank | 8 | 50 | 150 | 1 | 0.13 | 500-700 | 8,000 |

The aeration tank 15 used in the Example is a 4-chamber type. To maintain the MLSS (Mixed Liquor Suspended Solid: mean suspended substance concentration of mixing solution in aeration tank of each chamber in higher level of concentration, the total sludge are returned to first chamber 16 of aeration tank 15 at the returned rate of 150∼500% to treated amount of flowing water: a) sludge of inner circulation chamber 20 at the returned rate of 100∼300%, and b) sludge of final sedimentation tank 24 at the returned rate of 50∼200% to treated amount of flowing water.

Further, a small amount of sludge (40m³/day) from inner circulation chamber 20 of aeration tank 15 is returned to pretreatment facilities 2. To ensure the survival environment of Bacillus, a target microorganism for the predominant occupation, from the initial process step and facilitate the cultivation of Bacillus in first chamber 16 of aeration tank 15, the daily supply amounts of silicide and magnesium compound are 12kg and 6kg, respectively.

The significant increase of returned rate in sludge plays a role to extend the survival of Bacillus against other microbes, together with tapered aeration in aeration tank 15.

The water discharged from aeration tank 15 is separated in the form of solid and liquid from final sedimentation tank 24 separation and its supernatant was discharged as it was or in case where it is necessary to treat phosphorus, etc. contained the supernatant in a lower concentration, the supernatant is under a chemical flocculent settling process for final discharge.

Further, raw-sludge 13, so generated from primary sedimentation tank 11, is treated from aerobic sludge digestion tank 38 with four-chamber type. To maintain MLSS of each chamber by about 8,000mg/l, the total sludges are returned to first chamber 40 of aeration tank 38 at the returned rate of 500∼700% to treated amount of flowing water: a) sludge of inner circulation chamber 44 of aerobic sludge digestion tank 38 at the returned rate of 200∼300%, b) sludge of sedimentation tank for aerobic sludge digestion tank 48 at the returned rate of 100%, and c) sludge of final sedimentation tank 24 at the returned rate of 200∼300% to treated amount of flowing water.

To facilitate the cultivation of Bacillus in first chamber 40 of aerobic sludge digestion tank 38, the daily supply amounts of silicide and magnesium compound are 0.6kg and 0.3kg, respectively.

As described in the above, the significant increase of returned rate in sludge plays a role to extend the survival of Bacillus against other microbes, together with tapered aeration in aerobic sludge digestion tank 38.

The water 49 separated from sedimentation tank for aerobic sludge digestion tank 48 is supplied to pretreatment facilities 2 for retreatment, and excess sludge 51 is dehydrated from sludge hydroextractor 54 and discharged in the form of sludge cake 55. Table 3 illustrates appearance of treated water per process.

**[TABLE 3]**

| | Suspended Solid (SS) | Biochemical Oxygen Demand (BOD) | Chemical Oxygen Demand (COD) | Total Nitrogen (T-N) | Total Phosphorus (T-P) |
|---|---|---|---|---|---|
| Raw Water | 180 | 200 | 160 | 40 | 8 |
| Treated Water of Aeration Tank | 15 | 10 | 15 | 5 | 2 |
| Treated Water of Chemical Flocculent Settling | 5 | 5 | 10 | 5 | Trace |

As shown in table 3 on the treatment efficiency of each process, it is noted that the elimination efficiency of BOD and SS in flowing sewage is enhanced, while phosphorus is completely eliminated.

The aeration amount for dissolved oxygen in aeration tank 15 according to the Example embodying this invention is as follows:
- The first chamber 16 : 0.6m³/hr per unit volume(1m³);
- The second to fourth chambers 17 18 19 : 0.1m³/hr per unit volume(1m³), respectively.

The aeration amount for dissolved oxygen in aerobic sludge digestion tank 38 is as follows:
- The first chamber 40 : 1m³/hr per unit volume(1m³);
- The second to fourth chambers 41, 42, 43 : 0.2m³/hr per unit volume(1m³), respectively.

Further, a supervisory monitoring system 35 is used to observe the microorganism so as to control the aeration amount, internal circulation amount and sludge-returned amount and determine the dose of enhancer.

Namely, as shown in the following table 4, phase contrast microscope, a supervisory monitoring system 35 comprising camera, monitor, videoset and printer is used to observe the state of microorganisms present in aeration tank 15, aerobic sludge digestion tank 38 and various sludges returned and its results are utilized for operating the system and determining various influential factors.

The offensive-odoring gases 4, 8, 9 in high concentration, so generated from pretreatment facilities 2, flow control tank 5 and primary sedimentation tank 11, is supplied to first chamber 16 of aeration tank 15. When these gases are disintegrated by Bacillus, offensive-odoring gases are nearly disappeared in aeration tank 15, aerobic sludge digestion tank 38 and sludge storage tank 52 including each of sedimentation tank 24, 48. Table 4 illustrates the comparison between the method for treating the sewage-water and conventional method.

**[TABLE 4]**

| | Microorganism Occupied Predominantly | Aeration Method | Sludge Return | Supervisory Monitering System | Deodoriza-tion method | Chemicals |
|---|---|---|---|---|---|---|
| Method embodying Invention | Bacillus | Tapered Aeration | Aeration Tank: 1.5∼5Q | Available System | Deodoriza-tion by Bacillus (Aeration Tank) | -Enhancer for Microorganism |
| | | | Aerobic Sludge Digestion Tank: 5∼7Q | | | -Polymer Coagulant |
| Conventional Method | Various type of aerobic microorganism | Uniformity Aeration | Aeration Tank: 0.3∼0.6Q | Non-available System | -Incineration | -Polymer Coagulant |
| | | | | | -Washing by Chemicals | |
| | | | Aerobic Sludge Digestion Tank: 0.3Q | | | |
| | | | | | -Adsorption by Activated carbon | |
| | | | | | - Soil deodorizat-ion | |

In the table 4, the aeration tank of 1.5∼5Q includes the individual of 1∼3Q and the final sedimentation tank of 0.5∼2Q. Also, the aerobic sludge digestion tank of 5∼7Q includes the individual of 2∼3Q, the aerobic sludge digestion sedimentation tank of 1Q and the final sedimentation tank of 2∼3Q.

As described in the above, embodiments of this invention have some advantages as follows:

Since the metabolic action of Bacillus contributes much to eliminating nitrogen and phophorous as well as organic substances contained in sewage and sludges precipitate from the sewage, the BOD of discharged water may be maintained at 10mg/l with total nitrogen contained in the ater at less than 5mg/l and total phosphorus at less than 2mg/l.

Further, the precipitation of sludge is significantly enhanced; the sewage-water treatment is well performed in aeration tank and aerobic sludge digestion tank where loading change of organic substances having relatively great change or outer environmental changes exist; since offensive-odoring gases may be guided into aeration tank without separate facilities such as incinerator or adsorption tower, bad odors may be effectively eliminated.

Further, since sludges generated after the sewage-water treatment of this invention is the ones where Bacillus is predominantly occupied, far less amount of chemicals owing to remarkable dehydration is required in treatment over the conventional methods or no chemicals are required in embodiments of this invention. In comparison with the conventional system for treating sewage-water, relatively less sludge cake is generated and such cake may become superior compost.

Further, according to embodiments of this invention, a supervisory monitoring system is used to continuously observe the state of microorganisms such as protozoas including bacteria containing Bacillus in aeration tank and aerobic digestion tank and its observation results may be utilized for performing physicochemical operation and controlling various biological influential factors, thus optimizing the treatment conditions, maintain the microorganisms in an optimum level with easy and simple operation.

## Claims

1. A system for treating sewage-water comprising:
pretreatment facilities (2) for eliminating adulteration, microsome, or uncorrosive inorganic substance contained in the sewage-water supplied from a conduit;
a flow control tank for controlling the amount of water flowing from said pretreatment facilities (2);
a primary sedimentation tank (11) where water (6), so supplied from said flow control tank (5), is settled for a certain period of time and more than 5µ of suspended solid is precipitated;
an aeration tank (15) where water (12), so supplied from said primary sedimentation tank (11) is aerially treated and at the same time, the predominantly occupied cultivation of Bacillus is made;
a final sedimentation tank (24) where water (23), so supplied from said aeration tank (15), is settled for a certain period of time and its solid/liquid separation occurs by gravity;
an aerobic sludge digestion tank (38) where raw-sludge (13), so precipitated from said primary sedimentation tank (11), including sludge returned after being precipitated in said final sedimentation tank (24), are supplied for aerial treatment and at the same time, the predominantly occupied cultivation of Bacillus is selectively made;
a sedimentation tank (48) for aerobic sludge digestion tank where water (46), so discharged from said aerobic sludge digestion tank (38) is supplied and settled for a certain period of time and the precipitation of sludge contained in water (46) occurs;
chemical flocculent settling facilities (27) where water (25), so supplied from said sedimentation tank (24), is adequately treated to meet the discharge standards;
a sludge thickener (30) where remaining excess sludge (29) unreturned after precipitation in said final sedimentation tank (24) is concentrated;
a sludge storage tank (52) where remaining excess sludge (29) unreturned after precipitation in said sedimentation tank (48) for aerobic sludge digestion tank as well as sludge (32) concentrated from sludge thickener (30) are reserved;
a supervisory monitoring system (35) where some microorganisms such as bacteria, and protozoa present in said aeration tank (15), final sedimentation tank (24), aerobic sludge digestion tank (38) and sedimentation tank for aerobic sludge digestion tank (48) are under closer observation in order for its results to be utilized for controlling various influencing factors; and
a sludge hydroextractor (54) where sludge (53) is dehydrated, so discharged from said sludge storage tank (52).

2. A system for treating sewage-water according to claim 1, wherein each of said aeration tank (15) and aerobic sludge digestion tank (38) are separated and partitioned by a plurality of chambers, each of which has an aerator, and wherein said aeration tank (15) at the discharging part has an inner circulation chambers (20,44) designed to return sludges to the first chamber.

3. A system for treating sewage-water according to claim 1 or 2, wherein some mixing liquid from said aeration tank (15) and inner circulation chamber (20) is circulated to pretreatment facilities (2) in order that Bacillus may be grown in pretreatment facilities (2) and at the same time, bad odors may be eliminated.

4. A system for treating sewage-water according to claim 1, 2 or 3, wherein sludge-separated liquid (31), so generated from said sludge thickener (30) and the water, so generated from said sedimentation tank for aerobic sludge digestion tank (48), are resupplied to pretreatment facilities (2).

5. A system for treating sewage-water according to claim 1, 2, 3 or 4, wherein said supervisory monitoring system (35) comprises:
a phase contrast microscope designed to observe microorganisms;
a camera designed to photograph some microorganisms observed under said phase contrast microscope;
a videoset designed to investigate the microorganisms photographed by said camera;
a monitor designed to display microorganisms related information reproduced by said videoset; and
a printer designed to print microorganisms related information displayed on said monitor.

6. A method to treat sewage-water using pretreatment facilities, flow control tank, primary sedimentation tank, aeration tank, final sedimentation tank, aerobic sludge digestion tank, sedimentation tank for aerobic sludge digestion tank, sludge thickener, sludge storage tank and sludge hydroextractor, said method comprising the steps of:
allowing the amounts of total air, being charged to the aeration tank (15) and aerobic sludge digestion tank, to be aerated by gradation to first chambers and remaining second chambers in a certain ratio, respectively;
providing nutrients for cultivation of Bacillus into said aeration tank and aerobic sludge digestion tank;
allowing the internal circulation of sludge to be available between each inner circulation chamber and first chambers, so formed in both said aeration tank and aerobic sludge digestion tank;
allowing some sludge deposited from said final sedimentation tank to be returned and supplied to said first chamber of said aeration tank; and
allowing some sludges deposited from said sedimentation tank for aerobic sludge digestion tank and final sedimentation tank, respectively, to be returned and supplied to said first chamber of said aerobic sludge digestion tank.

7. A method to treat sewage-water according to claim 6, wherein nutrient for cultivation of Bacillus supplied to said aeration tank and aerobic sludge digestion tank comprises silicide and magnesium compound in order to facilitate the proliferation of fungus and induce its spores.

8. A method to treat sewage-water according to claim 6 or 7, wherein the sludge return rate of internal circulation of said aeration tank is in the range of 100∼300% to treated amount of flowing water, while the sludge return rate of internal circulation of said aerobic sludge digestion tank is in the range of 200∼300% to treated amount of flowing water.

9. A method to treat sewage-water according to claim 6, 7 or 8, wherein the rate of sludge returned from the outside to said aeration tank is in the range of 50∼200% to treated amount of flowing water, while the returned rate of sludge in the internal circulation of said aerobic sludge digestion tank is in the range of 200∼300% to treated amount of flowing water.

10. A method to treat sewage-water according to any of claims 6 to 9, wherein the total returned rate of sludge supplied to the first chamber of said aeration tank is in the range of 150∼500% to treated amount of flowing water, while the total returned rate of sludge supplied to the first chamber of said aerobic sludge digestion tank is in the range of 500∼700% to treated amount of flowing water.

11. A method to treat sewage-water according to any of claims 6 to 10, wherein the treated amount of flowing water, 1∼3% aeration tank circulation water from an inner circulation chamber of said aeration tank is transferred to said pretreatment facilities.

12. A method to treat sewage-water according to any of claims 6 to 11, wherein in case of the aeration amount of each first chamber provided into said aeration tank and aerobic sludge digestion tank, 60-80% of the total air amount supplied to both said aeration tank and aerobic sludge digestion tank is intensively given, while 20∼40% of the total air amount is aerated in small amount to the remaining chambers based on uniformity allocation.

13. A method to treat sewage-water according to any of claims 6 to 12, wherein offensive-odoring gases, so generated from said pretreatment facilities, flow control tank and primary sedimentation tank, are collected and transferred to said aeration tank where Bacillus is cultivated and predominantly occupied.

14. A method to treat sewage-water according to any of claims 6 to 13, wherein raw-sludge, so precipitated from said primary sedimentation tank, is aerobically digested using Bacillus.

15. A method to treat sewage-water according to claim 7 or any of claims 8 to 14 when appended thereto, wherein the dose of silicide is in the range of 0.01∼0.1kg/kg·BOD, while that of said magnesium compound is in the range of 0.005∼0.05kg/kg·BOD.
